# EUROPEAN PATENT APPLICATION

(11) **EP 4 729 085 A1**
(43) Date of publication of application: **22.04.2026**
(21) Application number: 24207525.7
(22) Date of filing: 18.10.2024
(51) Int. Cl.: A61M 5/14, A61M 5/142, A61M 5/145

(54) **INFUSION PUMP UNIT**

(71) Applicant: Syai UK Ltd, Cambridge CB2 8DL (GB)
(72) Inventor: Wang, Zhihua, Cambridge, CB2 8DL (GB)
(74) Representative: Nederlandsch Octrooibureau

(57) **Abstract**

The present application relates to the technical field of medical equipment, discloses an infusion pump unit comprising a case, a needle module and a drug delivery module. The case comprises a bottom case assembly and a front case assembly, and the needle module comprises a needle support, a metal catheter for drug delivery, a flexible indwelling hose, a first support, a second support, a detection spring piece and a reset assembly; the needle support may be movably penetrated to the front case assembly along a first direction, and the flexible indwelling hose is sleeved at one end of the metal catheter for drug delivery. The metal catheter for drug delivery is fixed to the first support, and the flexible indwelling hose is fixed to the second support; the detection spring piece is fixed to the bottom case assembly, and the second support is clamped with the detection spring piece when the flexible indwelling hose is located at a drug injection site. The reset assembly is used to drive the metal catheter for drug delivery to move along a direction opposite to the first direction relative to the flexible indwelling hose. The drug delivery module is used to store drugs and deliver drugs through the metal catheter for drug delivery. The above infusion pump unit can reduce the product size.

## Description

### Technical Field

The present application relates to the technical field of medical equipment, and particularly to an infusion pump unit.

### Background

At present, some existing infusion pumps are configured on belts, carried in pockets, or adhered to the skin in a patch-like manner. During use of the above infusion pump, users inject the drugs vertically into their skin through a metal injection infusion tube. However, the existing metal injection infusion tube cannot be withdrawn after vertically entering the skin, causing users to feel foreign objects and pain.

To solve the above problem that the metal injection infusion tube cannot be withdrawn from the skin, the existing infusion pump is provided with an indwelling needle, which is buried into the user's skin for injection. Then, in order to allow the indwelling needle to pierce the user's skin to a sufficient depth, the above-mentioned infusion pump often increases the travel depth of the indwelling needle enough, resulting in an increase in the size of the product and making it inconvenient for users to carry.

### Summary

The present application provides an infusion pump unit that not only solves the problem on withdrawal of the metal catheter, but also reduces the size of the infusion pump unit.

The present application provides an infusion pump unit, comprising a case, a needle module and a drug delivery module;
the case comprises a bottom case assembly and a front case assembly, and the front case assembly is covered on the bottom case assembly;
the needle module comprises a needle support, a metal catheter for drug delivery, a flexible indwelling hose, a first support, a second support, a detection spring piece and a reset assembly located within the case;
the needle support may be movably penetrated to the front case assembly along a first direction relative to the front case assembly, and the first direction is the orientation of the front case assembly and the bottom case assembly;
the flexible indwelling hose is extended along the first direction, sleeved at one end of the metal catheter for drug delivery, and in interference fit with the metal catheter for drug delivery;
the metal catheter for drug delivery is fixed to the first support, and the flexible indwelling hose is fixed to the second support; the detection spring piece is fixed to the bottom case assembly to drive the first support along the first direction wen the needle support moves along the first direction, so that part of the metal catheter for drug delivery located in the flexible indwelling hose and the flexible indwelling hose are movably penetrated to the bottom case assembly along the first direction, and the second support is clamped with the detection spring piece when the flexible indwelling hose is located at a drug injection site;
the reset assembly is used to drive part of the metal catheter for drug delivery located in the flexible indwelling hose to move along a direction opposite to the first direction relative to the flexible indwelling hose when the flexible indwelling hose is located at the drug injection site;
the drug delivery module is used to store drugs and deliver drugs through the metal catheter for drug delivery.

For the infusion pump unit provided by the present application, a first support, a second support, a detection spring piece and a reset assembly are provided in the case, and the metal catheter for drug delivery is sleeved on the flexible indwelling hose. When injecting, the user can use the needle support to push the first support to move in the first direction, so that the metal catheter for drug delivery is pierced into the user's skin with the flexible indwelling hose. When the second support is clamped with the detection spring piece, the needle support is released, and the reset assembly can drive the metal catheter for drug delivery to detach from the user's skin, while the flexible indwelling hose is buried in the skin. Then, the drug delivery module guides the drugs into the flexible indwelling hose through the metal catheter for drug delivery and finally into the user's body. Provision of a reset assembly for the infusion pump unit allows the metal catheter for drug delivery to detach from the skin during the injection process, without causing pain to the user. Provision of a detection spring piece can accurately detect the depth of the metal catheter for drug delivery piercing into the skin, which is conducive to controlling the overall height of the product and avoiding the problem of enlarging the product size in the prior art to ensure sufficient depth. Therefore, it is beneficial to reduce the size of the product, making it inconvenient for users to carry.

In some possible embodiments, the reset assembly comprises a reset spring, one end of which is connected to the bottom case assembly, and the other end is connected to the first support.

In some possible embodiments, the reset assembly comprises a guide shaft, which is extended along the first direction; the guide shaft is fixed to the bottom case assembly, and the reset spring is sleeved on the guide shaft.

In some possible embodiments, the first support is made of metal, and the second support is also made of metal.

In some possible embodiments, it further comprises an alarm buzzer provided in the case;
the number of the detection spring pieces is two, with a spacing between the two detection spring pieces, and the detection spring pieces are made of metal;
the alarm buzzer is electrically connected with one of the detection spring pieces, so as to drive the alarm buzzer to give an alarm when the second support is clamped with the two detection spring pieces.

In some possible embodiments, the drug delivery module comprises a cartridge and a drive device, wherein the cartridge is used for storing drugs, and the drive device is used to drive the drugs in the cartridge to flow to the metal catheter for drug delivery.

In some possible embodiments, the drug delivery module further comprises a filter assembly, and the filter assembly comprises a filtering container connected to the cartridge and a first filter layer, a second filter layer and a filtering silicone sealant plug located inside the filtering container and arranged sequentially along the flow direction of the drugs from the cartridge to the metal catheter for drug delivery, wherein the metal catheter for drug delivery is penetrated to the filtering silicone sealant plug; the first filter layer is used to filter particle impurities with a diameter φ of 100µm-1mm, and the second filter layer is used to filter particle impurities with a diameter φ of 0.2µm-100µm.

In some possible embodiments, the driving assembly comprises a motor, a transmission assembly, and a piston;
one end of the transmission assembly is connected to the motor, and the other end is connected to the piston, so that the motor drives the piston to move towards the inside of the cartridge through the transmission assembly.
one end of the transmission assembly is connected to the motor, and the other end is connected to the piston, so that the motor drives the piston to move towards the inside of the cartridge through the transmission assembly.

In some possible embodiments, it further comprises a control unit and a signal recognition unit, wherein the signal recognition unit is electrically with the control unit, and the control unit is electrically with the motor;
the signal recognition unit is used to recognize signals of injection drugs, and the control unit controls the operation of the motor based on the signals recognized by the signal recognition unit.

In some possible embodiments, the front case assembly comprises a front case and a sealing cover.
the front case is provided with a connection, and the needle support can be detachably penetrated to the connection;
the sealing cover is connected to the front case, and the sealing cover is used to be closed to the connection when the needle support is detached from the connection.

### Brief Description of the Drawings

FIG. 1 is a schematic diagram of the overall structure of the infusion pump unit in the embodiment of the present application;
FIG. 2 is an exploded view of the infusion pump unit in the embodiment of the present application;
FIG. 3 is a schematic diagram of a cross-sectional structure at the needle module in the embodiment of the present application;
FIG. 4 is a schematic diagram of a cross-sectional structure of the flexible indwelling hose located at the drug injection site in FIG. 3;
FIG. 5 is a schematic diagram of another cross-sectional structure of the flexible indwelling hose located at the drug injection site in FIG. 3;
FIG. 6 is a schematic diagram of a cross-sectional structure of the first support in FIG. 3 when it returns to the initial position;
FIG. 7 is a structural diagram of the inside of the infusion pump unit in the embodiment of the present application;
FIG. 8 is an exploded view of the drug delivery module in the embodiment of the present application;
FIG. 9 is a schematic diagram of a partial cross-sectional structure of the drug delivery module in the embodiment of the present application;
FIG. 10 is a schematic diagram of another partial cross-sectional structure of the drug delivery module in the embodiment of the present application;
FIG. 11 is a schematic diagram of a cross-sectional structure of the metal catheter for drug delivery in the embodiment of the present application; and
FIG. 12 is another yet schematic diagram of the overall structure of the infusion pump unit in the embodiment of the present application.

In the drawings:
100-case; 110-front case assembly; 111-front case; 1111-connection; 1112-sealing cover; 112-side plate; 120-bottom case assembly; 200-needle module; 210-needle support; 220-metal catheter for drug delivery; 230-flexible indwelling hose; 240-first support; 250-second support; 260-detection spring piece; 270-reset assembly; 271-reset spring; 272-guide shaft; 300-drug delivery module; 310-cartridge; 311-opening; 320-drive device; 321-motor; 322-leadscrew; 323-push rod loose piece; 324-piston; 330-filter assembly; 331-filtering container; 332-first filter layer; 333-second filter layer; 3331-filtering membrane; 3332-positioning ring; 334-filtering silicone sealant plug; 335-annular butt joint; 400-alarm buzzer; 500-power module; 600-signal recognition unit; 700-control unit.

### Detailed Description of the Preferred Embodiments

Technical solutions in the embodiments of the present invention will be described clearly and completely in combination with figures in the embodiments of the invention. Obviously, the described embodiments are only part, but not all, of the embodiments of the invention. All other embodiments obtained by those of ordinary skill in the art without creative work based on the embodiments of the present invention are within the scope of protection of the present invention.

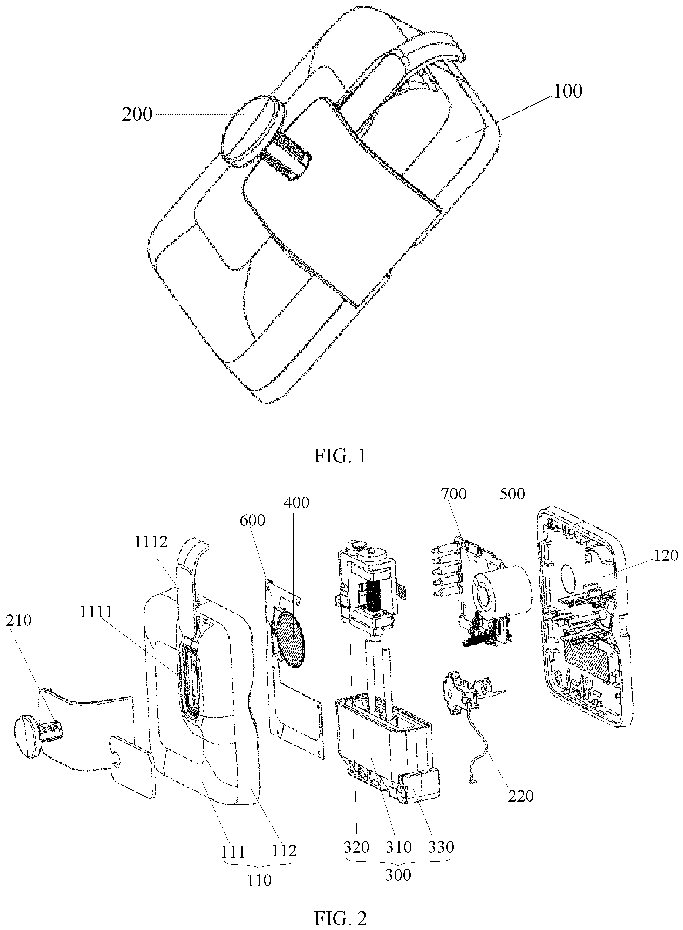

Referring to FIGs. 1 and 2, the infusion pump unit in the embodiment of the present application comprises a case 100, a needle module 200, and a drug delivery module 300, wherein the needle module 200 and the drug delivery module 300 are both provided on the case 100.

Specifically, the case 100 comprises a front case assembly 110 and a bottom case assembly 120, wherein the front case assembly 110 is covered on the bottom case assembly 120 to form a case 100 structure with internal accommodating space. As shown in FIG. 2, the front case assembly may comprise a front case 111 and a side plate 112 connected to the front case 111 around the front case. When the front case assembly 110 is covered on the bottom case assembly 120, the front case 111 is opposite to the bottom shell assembly 120, and the side plate 112 is used to connect the front case 111 and the bottom case assembly 120. At this time, the spacing between the front case 111 and the bottom case assembly 120 can be understood as the height of the case 100, and the orientation between the front case assembly 110 and the bottom case assembly 120 is set as the first direction, that is, the first direction is the height direction of the case 100, and the first direction is perpendicular to the bottom case assembly 120.

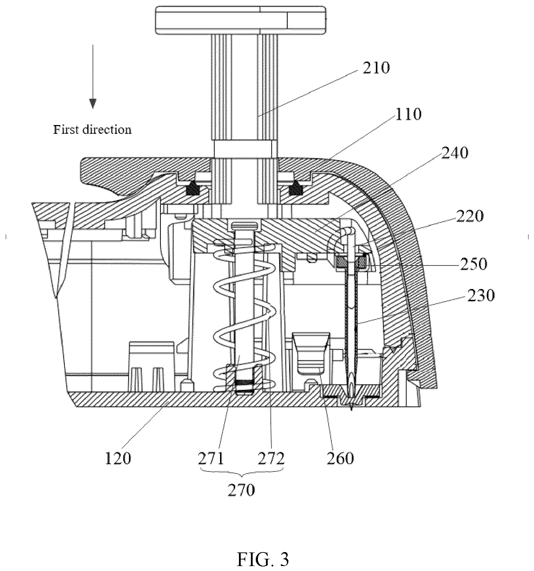

Referring to FIGs. 2 and 3 together, the needle module 200 comprises a needle support 210, a metal catheter for drug delivery 220, a flexible indwelling hose 230, a first support 240, a second support 250, a detection spring piece 260, and a reset assembly 270. The front case 111 is provided with a connection 1111, which is used to connect the inside and outside of the case 100. The needle support 210 can be movably penetrated to the connection 1111 along the first direction relative to the front case 11, that is, one end of the needle support 210 is located inside the case 100 and the other end is located outside the case 100.

The metal catheter for drug delivery 220, flexible indwelling hose 230, first support 240, second support 250, detection spring piece 260 and the reset assembly 270 are all located inside of the case 100, wherein the first support 240 is fixedly connected with the metal catheter for drug delivery 220, and the flexible indwelling hose 230 is fixedly connected with the second support 250, so that the first support 240 and the second support 250 can play support roles in the metal catheter for drug delivery 220 and the flexible indwelling hose 230. The flexible indwelling hose 230 is extended along the first direction, and the flexible indwelling hose 230 can move along the first direction relative to the case 100. The flexible indwelling hose 230 is sleeved at one end of the metal catheter for drug delivery 220, and in interference fit with the metal catheter for drug delivery 220, that is, the metal catheter for drug delivery 220 and the flexible indwelling hose 230 can keep relatively fixed without external interference. It should also be noted that in the initial state, the end of the metal catheter for drug delivery 220 passes through the flexible indwelling hose 230 and is exposed to the flexible indwelling hose 230, so that the metal catheter for drug delivery 220 can be quickly pierced into the user's skin.

When the infusion pump unit in this embodiment is in its initial state, continue to refer to FIG. 3. The first support 240 is located near the front case 111, and one end of the needle support 210 located inside the case 100 can be in contact with one side of the first support 240 away from the bottom case assembly 120. When the needle support 210 moves along the first direction, it can drive the first support 240 to move synchronously along the first direction and drive the metal catheter for drug delivery 220 and the flexible indwelling hose 230 to move along the first direction. The bottom case assembly 120 is provided with a through hole, through which the flexible indwelling hose 230 and the metal catheter for drug delivery 220 can expose the surface of the bottom case assembly 120 during the movement along the first direction after passing through the bottom case assembly 120.

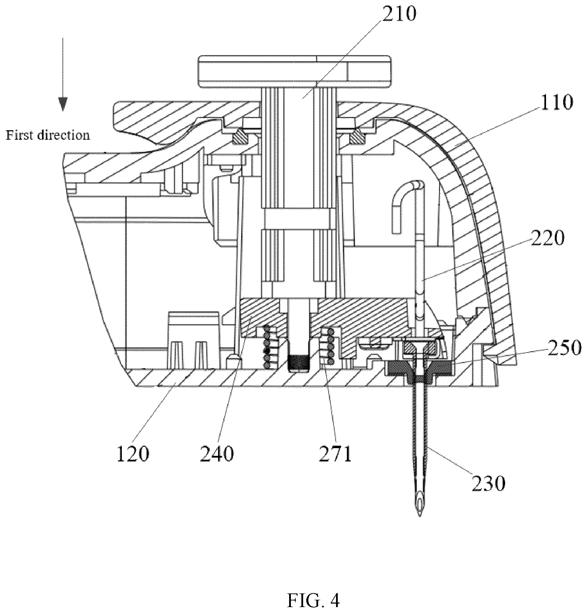

Referring to FIGs. 3 and 4 together, the detection spring piece 260 is fixed to the bottom case assembly 120. Along the first direction, the second support 250 is located between the first support 240 and the detection spring piece 260. When the flexible indwelling hose 230 moves along the first direction, the second support 250 moves toward the detection spring piece 260. When the flexible indwelling hose 230 moves to the drug injection site, the second support 250 can be clamped with the detection spring piece 260. At this time, the flexible indwelling hose 230 cannot move along the first direction or along a direction opposite to the first direction again. It should be noted that the drug injection site can be understood as the relative position between the flexible indwelling hose 230 and the bottom case assembly 120 when the portion of the flexible indwelling hose 230 and the metal catheter for drug delivery 220 passing out the bottom case assembly 120 is sufficient. At this time, the depth of the part of the flexible indwelling hose 230 and the metal catheter for drug delivery 220 located outside the case 100 entering the user's skin is sufficient to ensure effective injection effect.

The reset assembly 270 may comprise a reset spring 271 and a guide shaft 272, wherein the guide shaft 272 is extended along the first direction, and one end of the guide shaft 272 is fixed to the bottom case assembly 120; the reset spring 271 is sleeved on the guide shaft 272, and the reset spring 271 can stretch relative to the guide shaft 272 along the first direction. It can be understood that the reset spring 271 is sleeved on the guide shaft 272, which can guide the stretching of the reset spring 271, ensuring that the reset spring 271 undergoes deformation along the first direction. One end of the reset spring 271 is connected to the bottom case assembly 120, and the other end is connected to the first support 240. The first support 240 is also provided with a through groove that runs through along the first direction. The side of the needle support 210 facing the first support 240 is also provided with an avoidance slot. When the needle support 210 and the first support 240 move along the first direction, the end of the guide shaft 272 away from the bottom case assembly 120 can pass through the through groove and be located in the avoidance slot to avoid interference with the movement of the first support 240 by the guide shaft 272.

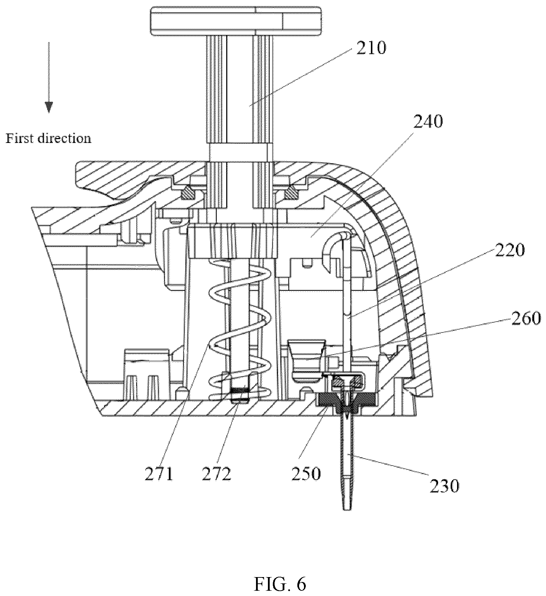

When the user pushes the needle support 210 toward the inside of the case 100, the first support 240 moves along the first direction, the reset spring 271 is compressed, and an elastic reset force is generated to reset the first support 240. Referring to FIG. 6, when the second support 250 is clamped with the detection spring piece 260, the thrust acting on the needle support 210 can be cancelled. Under the elastic reset force of the reset spring 271, the reset spring 271 drives the first support 240 to move along a direction opposite to the first direction, so that the first support 240 drives the part of the metal catheter for drug delivery 220 located in the flexible indwelling hose to move along the direction opposite to the first direction relative to the flexible indwelling hose 230. At this time, it can be ensured that only the flexible indwelling hose 230 is located inside the user's skin, while the metal catheter for drug delivery 220 is detached from the user's skin. During the subsequent injection of the drugs, only the flexible indwelling hose 230 is buried inside the user's skin, without causing pain or skin trauma to the user.

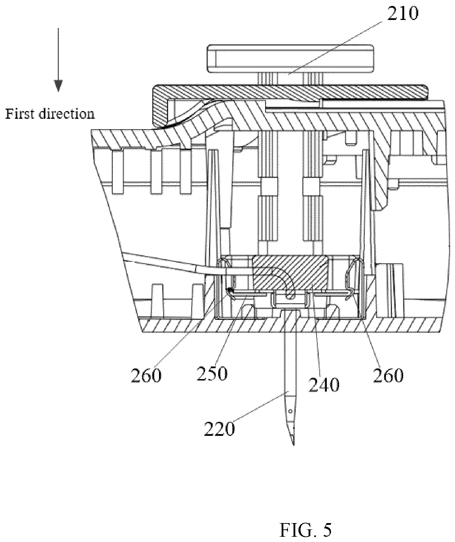

Referring to FIGS. 2 and 5 together, the infusion pump unit in this embodiment further comprises a power module 500 and an alarm buzzer 400 provided inside the case 100. The number of the detection spring pieces 260 is two, with a spacing between the two detection spring pieces 260, and the detection spring pieces 260 are made of metal. One of the detection spring pieces 260 is connected with the power module 500, while the other detection spring piece 260 is electrically connected with the alarm buzzer 400. Under normal circumstances, due to the lack of contact between the two detection spring pieces 260, the power module 500 does not supply power to the alarm buzzer 400, and the alarm buzzer 400 will not sound an alarm. The second support 250 is also made of metal. When the second support 250 moves to its two ends and is respectively clamped with two detection spring pieces 260, a circuit is formed between the two detection spring pieces 260. At this time, the power module 500 can supply power to the alarm buzzer 400, which can sound an alarm to remind the user that the metal catheter for drug delivery 220 and the flexible indwelling hose 230 have pierced into the user's skin to a sufficient depth for drug delivery.

In addition, the first support 240 in this embodiment can also be made of metal. It is worth noting that in this embodiment, the first support 240, the second support 250, and the detection spring piece 260 are all made of metal, so that the above components can be formed using sheet metal forming technology. Compared with the existing plastic support, the metal support can effectively reduce the vertical height (i.e. the size along the first direction), which is conducive to reducing the overall thickness of the product.

Referring to FIGs. 2, 7, and 8 together, the drug delivery module 300 in this embodiment comprises a cartridge 310, a drive device 320, and a filter assembly 330. The cartridge 310 can be used to store drugs, and the filter assembly 330 is provided between the cartridge 310 and the drug delivery metal conduit 220. The drive device 320 is used to drive the drugs in the cartridge 310 to flow to the metal catheter for drug delivery 220. Before the drug flows into the metal catheter for drug delivery 220, the drug can pass through the filter assembly 330, which is used to filter the drug, thereby filtering out impurities in the drug, which cannot only avoid blocking the metal catheter for drug delivery 200, but also can prevent impurities from entering the user's body.

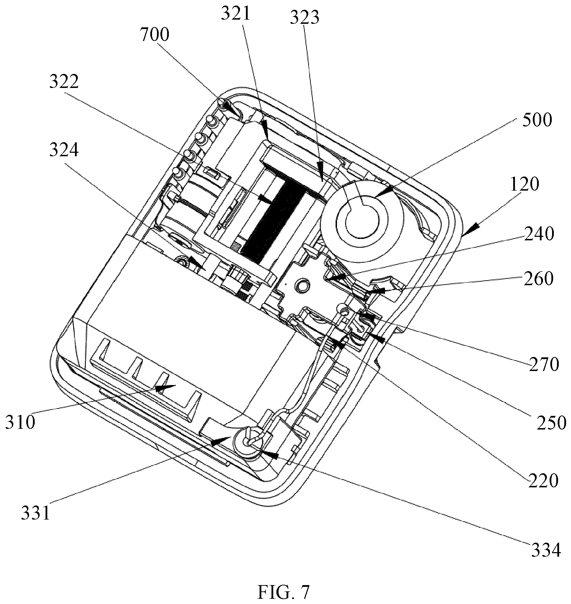

Specifically, as shown in FIG. 7, the drive device 320 may comprise a motor 321, a transmission assembly, and a piston 324. The transmission assembly may comprise, for example, a leadscrew 322 and a push rod loose piece 323. The leadscrew 322 is connected to the output shaft of the motor 321, and the power module 500 can supply power to the motor 321, so that the motor 321 can drive the leadscrew 322 to rotate around the axis of the leadscrew 322. The push rod loose piece 323 is connected to the leadscrew 322 in a transmission manner, and when the leadscrew 322 rotates, it can drive the push rod loose piece 323 to move relative to the leadscrew 322 along the axis direction of the leadscrew 322. The push rod loose piece 323 is also connected to the piston 324. When the push rod loose piece 323 moves along the axis of the leadscrew 322, it can drive the piston 324 to move synchronously along the axis of the leadscrew 322.

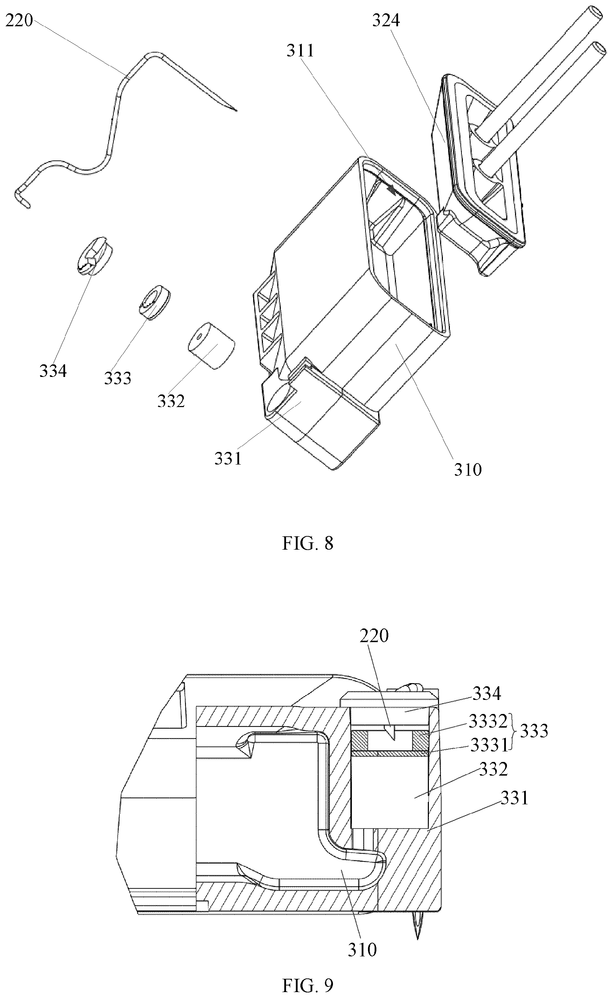

As shown in FIG. 8, an opening 311 is provided at one end of the cartridge 310 facing the drive device 320, and the sealing cover 1112 for the piston 324 is closed to the opening 311 to maintain a sealed environment inside the cartridge 310 with the cooperation of the piston 324. The drug is stored on the side of the cartridge 310 away from the piston 324. When the push rod loose piece 323 drives the piston 324 to move along the axis of the leadscrew 322, it can cause the piston 324 to move relative to the opening 311 of the cartridge 310 towards the inside of the cartridge 310 or away from the inside of the cartridge 310. The side of the cartridge 310 opposite to the opening 311 is provided with a drug outlet. When the piston 324 moves towards the interior of the cartridge 310, the piston 324 applies pressure to the drug inside the cartridge 310, allowing the drug to flow out from the drug outlet.

As shown in FIGs. 8 and 9, the filter assembly 330 comprises a filtering container 331 and a first filter layer 332, a second filter layer 333 and a filtering silicone sealant plug 334 located inside the filtering container 331, wherein the first filter layer 332, second filter layer 333, and the filtering silicone sealant plug 334 are sequentially arranged along the flow direction of the drug from the cartridge 310 to the metal catheter for drug delivery 220, and one end of the metal catheter for drug delivery 220 away from the flexible indwelling hose 230 is penetrated to the filtering silicone sealant plug 334 to ensure that the end of the metal catheter for drug delivery 220 is located in the filtering container 331. That is, when the drug flows out from the drug outlet of the cartridge 310, it sequentially passes through the first filter layer 332, second filter layer 333, and filtering silicone sealant plug 334 before entering the metal catheter for drug delivery 220. Moreover, after the first support 240 returns to its initial position, the end of the metal catheter for drug delivery 220 remains inside the flexible indwelling hose 230, and the metal catheter for drug delivery 220 remains relatively fixed with the flexible indwelling hose 230, so that the drug can be completely injected into the flexible indwelling hose 230 through the metal catheter for drug delivery 220, and finally injected into the user's skin.

In this embodiment, the first filter layer 332 can be used for initial filtration of the drug, with the particle impurities with a diameter φ of 100µm-1mm removed. The second filter layer 333 can perform secondary filtration on drugs, with the particle impurities with a diameter φ of 0.2µm-100µm removed. Under the blocking effect of the filtering silicone sealant plug 334, the drug cannot flow out of the filtering container 331 through other paths and can only flow out through the metal catheter for drug delivery 220.

Based on this, the material of the first filter layer 332 in this embodiment can be sponge or polyethylene, and the second filter layer 333 can include a filtering membrane 3331, or the material of the second filter layer 333 can also be polyethylene. When the first filter layer 332 or the second filter layer 333 is made of polyethylene, the structure for filtration can be made using the PE sintering process.

As shown in FIG. 9, when the second filter layer 333 includes a filtering membrane 3331, it also includes a positioning ring 3332. The positioning ring 3332 is located on the side of the filtering membrane 3331 away from the first filter layer 332, and both sides of the positioning ring 3332 are respectively abutted against the filtering silicone sealant plug 334 and the filtering membrane 3331, so as to make the filtering membrane 3331 fit against the first filter layer 332 and improve the filtering effect. The positioning ring 3332 and the filtering membrane 3331 can be an integrated structure to simplify the structure and facilitate assembly.

Due to the hollow structure in the middle of the positioning ring 3332, when the metal catheter for drug delivery 220 is penetrated into the filtering silicone sealant plug 334, the end of the metal catheter for drug delivery 220 can be extended into the hollow structure of the positioning ring 3332. After the drugs pass through the filtering membrane 3331, the hollow structure of the positioning ring 3332 can serve as a temporary storage for the drugs and allow the drugs to flow as much as possible into the metal catheter for drug delivery 220, thereby avoiding drug waste.

The secondary filtration method adopted in this embodiment can prevent large particle impurities from blocking the filtering membrane 333, thereby avoiding a decrease in the permeability rate of the filtering membrane 333 and preventing the reduction of drugs passing through the filtering membrane 333 from affecting the injection effect. In addition, the secondary filtration method can also reduce the usable area of the filtering membrane 333 and improve the filtration effect.

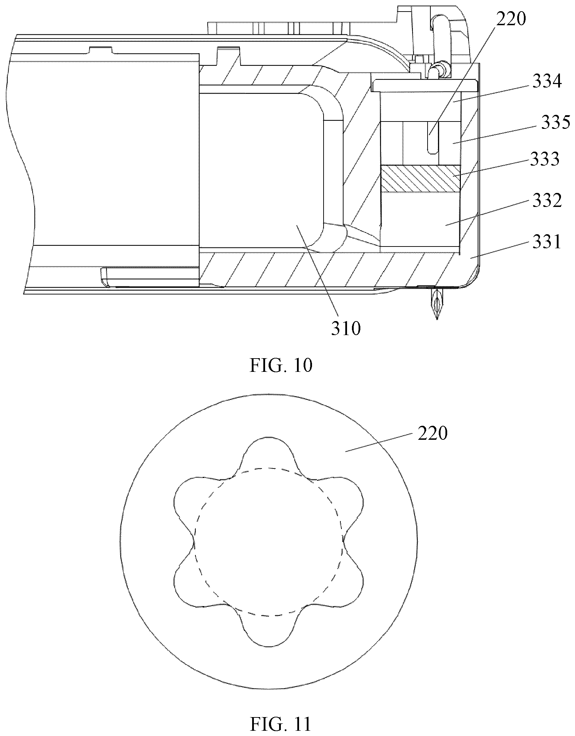

In some embodiments, referring to FIG. 10, when the second filter layer 333 is made of polyethylene, the side of the filtering silicone sealant plug 334 facing the second filter layer 333 is provided with an annular butt joint 335, and the side of the annular butt joint 335 facing the second filter layer 333 is abutted against the second filter layer 333 to ensure relative fixation between the filtering silicone sealant plug 334 and the second filter layer 333. Similarly, there is a hollow structure in the middle of the annular butt joint 335, and after the metal catheter for drug delivery 220 is penetrated into the filtering silicone sealant plug 334, the end of the metal catheter for drug delivery 220 can be extended into the hollow structure of the annular butt joint 335, in order to facilitate guidance of the drugs into the metal catheter for drug delivery 220.

The annular butt joint 335 and the filtering silicone sealant plug 334 can also be an integrated structure to simplify the structure and facilitate assembly.

As an optional embodiment, the filtering container 331 and the cartridge 310 may be integral, thus avoiding the introduction of secondary impurities and ensuring the quality of the drug.

In some embodiments, referring to Fi. 11, the cross-sectional shape of the metal catheter for drug delivery 220 penetrating into one end of the filtering silicone sealant plug 334 is a plum blossom shape. When the cross-sectional area is the same, compared to the circular shaped cross section, the diameter of the inner ring of the plum blossom shaped cross section is smaller than the diameter of the circular shaped cross section. When the drug contains smaller particle impurities, the drug can be further filtered by the metal catheter for drug delivery 220 with a plum blossom shaped cross section when entering the metal catheter for drug delivery 220.

Referring to FIG. 7 again, the cartridge 310, filter assembly 330, drive device 320, power module 500, and needle module 200, except for the needle support 210, are all arranged on the bottom case assembly 120, and the structural components of each part are laid independently and horizontally on the bottom case assembly 120. There is no stacking between any two structural components. On the one hand, the above structural components are compact in structure, and on the other hand, it is beneficial to reduce the vertical height of the infusion pump unit, thereby reducing the volume of the product.

Referring to FIG. 2 again, the infusion pump unit in this embodiment further comprises a signal recognition unit 600 and a control unit 700 provided inside the case 100. The signal recognition unit 600 may be an antenna assembly for communication reception and transmission, and the control unit 700 may be a circuit board. As mentioned earlier, the alarm buzzer 400 can remind the user that the flexible indwelling hose 230 is pierced into the skin in place. At this time, the user can, for example, operate on a mobile phone and generate a signal for injecting drugs. After receiving the signal, the signal recognition unit 600 sends the signal to the control unit 700. The control unit 700 can control the motor 321 to operate based on the received signal, thereby driving the piston 324 to move, so that the drug in the cartridge 310 flows towards the metal catheter for drug delivery 220. The power module 500 in this embodiment can also be used to supply power to the control unit 700 and the signal recognition unit 600 to ensure the normal operation of each electronic component.

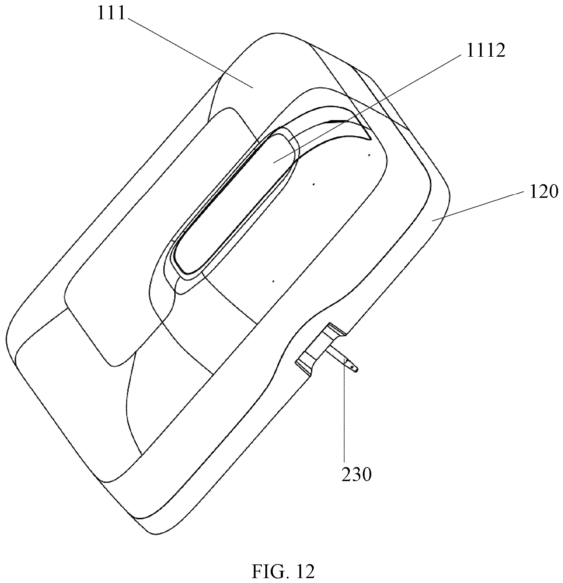

Referring to FIGs. 2 and 12 together, the needle module 200 is detachably connected with the front case 111, and the needle support 210 can be installed on the front case 111 when the flexible indwelling hose 230 is needed to pierce into the user's skin. The front case 111 is also connected to a sealing cover 1112, which can rotate or move relative to the front case 111, so that the sealing cover 1112 can be closed to the connection 1111 or expose the connection 1111. That is to say, when the infusion pump unit in this embodiment is not in use, the sealing cover 1112 is closed to the connection 1111 to ensure that the interior of the case 100 is isolated from the outside and to prevent impurities from entering the interior of the case 100. When it is necessary to inject drugs, the sealing cover 1112 can be opened first, the needle support 210 can be installed on the connection 1111, and the flexible indwelling hose 230 can be pierced. After the flexible indwelling hose 230 is pierced into the skin in place, the needle support 210 is removed, and then the connection 1111 is covered by the sealing cover 1112 to ensure that the inside of the case 100 is isolated from the outside.

It should be understood that for the infusion pump unit in this embodiment, the needle support 210 can be regarded as a universal component, that is to say, the needle support 210 can be produced and sold separately. In addition, the needle support 210 is detachable and can further reduce the volume of the infusion pump unit when not in use, making it convenient for users to carry.

Based on the introduction of the infusion pump unit in this embodiment, the specific process for using the infusion pump unit can refer to the following: first, open the sealing cover 1112 and install the needle support 210 on the connection 1111. Then, fit the outer surface of the bottom case assembly 120 against the surface of the user's skin, press the needle support 210, so that the metal catheter for drug delivery 220 pierces into the user's skin, allowing the flexible indwelling hose 230 to enter the user's skin. When the metal catheter for drug delivery 220 is pierced in place, the second support 250 is clamped with the detection spring piece 260, and the alarm buzzer 400 sounds an alarm. Thereafter, the user releases the needle support 210, and the reset spring 271 drives the metal catheter for drug delivery 220 to detach from the flexible indwelling hose 230, so that the metal catheter for drug delivery 220 is withdrawn from the user's skin and returns to the case 100. The flexible indwelling hose 230 is buried inside the user's skin. Next, the user will remove the needle support 210, and the sealing cover 1112 is closed to the connection 1111, to prevent particulate matter from entering the interior of the case 100. Next, the user uses the signal recognition module to send a signal for drug injection. The control unit 700 drives the motor 321 to operate based on the signal, causing the piston 324 to push the drug in the cartridge 310 to flow into the metal catheter for drug delivery 220. The drug is guided into the flexible indwelling hose 230 through the metal catheter for drug delivery 220, thereby entering the user's body.

The infusion pump unit in the embodiment of the present application ensures that the bottom case assembly 120 always fits against to the user's skin during the injection process, thereby achieving vertical injection. Moreover, the metal catheter for drug delivery 220 is withdrawn from the user's skin during the injection process, and the user will not experience any foreign body sensation or pain. In addition, the depth of the metal catheter for drug delivery 220 piercing into skin is detected using the detection spring piece 260, in order to ensure sufficient depth of piercing into skin, without excessively increasing the size of the product in the vertical injection direction, thereby reducing the product volume and making it inconvenient for users to carry.

It will be apparent to those skilled in the art that various amendments and variations may be made to the present invention without departing from the spirit and scope of the present invention. In this way, if these amendments and variations of the present invention are within the ranges of the claims of the present invention and equivalent technologies thereof, the present invention has also intended to contain those amendments and modifications.

## Claims

1. An infusion pump unit, **characterized by** comprising a case, a needle module and a drug delivery module, wherein
the case comprises a bottom case assembly and a front case assembly, and the front case assembly is covered on the bottom case assembly;
the needle module comprises a needle support, a metal catheter for drug delivery, a flexible indwelling hose, a first support, a second support, a detection spring piece and a reset assembly located within the case;
the needle support is configured to movably penetrate the front case assembly along a first direction relative to the front case assembly, and the first direction is the orientation of the front case assembly and the bottom case assembly;
the flexible indwelling hose is extended along the first direction, sleeved at one end of the metal catheter for drug delivery, and in interference fit with the metal catheter for drug delivery;
the metal catheter for drug delivery is fixed to the first support, and the flexible indwelling hose is fixed to the second support; the detection spring piece is fixed to the bottom case assembly configured to drive the first support along the first direction wen the needle support moves along the first direction, so that part of the metal catheter for drug delivery located in the flexible indwelling hose and the flexible indwelling hose are configured to movably penetrate the bottom case assembly along the first direction, and the second support is configured to be clamped with the detection spring piece when the flexible indwelling hose is located at a drug injection site;
the reset assembly is configured to drive part of the metal catheter for drug delivery located in the flexible indwelling hose to move along a direction opposite to the first direction relative to the flexible indwelling hose when the flexible indwelling hose is located at the drug injection site;
the drug delivery module is configured to store drugs and deliver drugs through the metal catheter for drug delivery.

2. The infusion pump unit according to claim 1, **characterized in that**, the reset assembly comprises a reset spring, one end of which is connected to the bottom case assembly, and the other end is connected to the first support.

3. The infusion pump unit according to claim 2, **characterized in that**, the reset assembly comprises a guide shaft, which is extended along the first direction; the guide shaft is fixed to the bottom case assembly, and the reset spring is sleeved on the guide shaft.

4. The infusion pump unit according to claim 1, **characterized in that**, the first support is made of metal, and the second support is also made of metal.

5. The infusion pump unit according to claim 4, **characterized by** further comprising an alarm buzzer provided in the case;
the number of the detection spring pieces is two, with a spacing between the two detection spring pieces, and the detection spring pieces are made of metal;
the alarm buzzer is electrically connected with one of the detection spring pieces, wherein the alarm buzzer is configured to give an alarm when the second support is clamped with the two detection spring pieces.

6. The infusion pump unit according to claim 1, **characterized in that**, the drug delivery module comprises a cartridge and a drive device, wherein the cartridge is configured to store drugs, and the drive device is configured to drive the drugs in the cartridge to flow to the metal catheter for drug delivery.

7. The infusion pump unit according to claim 6, **characterized in that**, the drug delivery module further comprises a filter assembly, and the filter assembly comprises a filtering container connected to the cartridge and a first filter layer, a second filter layer and a filtering silicone sealant plug located within the filtering container and arranged sequentially along the flow direction of the drugs from the cartridge to the metal catheter for drug delivery, wherein the metal catheter for drug delivery penetrates and is fixed to the filtering silicone sealant plug; the first filter layer is configured to filter particle impurities with a diameter φ of 100µm-1mm, and the second filter layer is used to filter particle impurities with a diameter φ of 0.2µm-100µm.

8. The infusion pump unit according to claim 6, **characterized in that**, the driving assembly comprises a motor, a transmission assembly, and a piston;
one end of the transmission assembly is connected to the motor, and the other end is connected to the piston, so that the motor is configured to drive the piston to move towards the inside of the cartridge through the transmission assembly.
one end of the transmission assembly is connected to the motor, and the other end is connected to the piston, so that the motor is configured to drive the piston to move towards the inside of the cartridge through the transmission assembly.

9. The infusion pump unit according to claim 8, **characterized by** further comprising a control unit and a signal recognition unit, wherein the signal recognition unit is electrically connected with the control unit, and the control unit is electrically connected with the motor;
the signal recognition unit is configured to recognize signals of injection drugs, and the control unit is configured to control the operation of the motor based on the signals recognized by the signal recognition unit.

10. The infusion pump unit according to claim 1, **characterized in that**, the front case assembly comprises a front case and a sealing cover;
the front case is provided with a connection, and the needle support is configured to detachably penetrate and be fixed to the connection;
the sealing cover is connected to the front case, and the sealing cover is used to be closed to the connection when the needle support is detached from the connection.
